(19) Europäisches Patentamt
European Patent Office
Office européen des brevets

(11) EP 1 303 261 B1

(12) **EUROPEAN PATENT SPECIFICATION**

(45) Date of publication and mention of the grant of the patent:
**09.03.2005 Bulletin 2005/10**

(21) Application number: **01954879.1**

(22) Date of filing: **20.07.2001**

(51) Int Cl.[7]: **A61K 9/48**, A61K 9/107

(86) International application number:
**PCT/US2001/023140**

(87) International publication number:
**WO 2002/007712 (31.01.2002 Gazette 2002/05)**

(54) **SELF-EMULSIFYING DRUG DELIVERY SYSTEMS FOR EXTREMELY WATER-INSOLUBLE, LIPOPHILIC DRUGS**

SELBSTEMULGIERENDES SYSTEM ZUR ABGABE VON SEHR WASSERUNLÖSLICHEN UND LIPOPHILEN ARZNEIMITTELN

SYSTEMES AUTO-EMULSIFIANT D'ADMINISTRATION DE MEDICAMENTS LIPOPHILES EXTREMEMENT INSOLUBLES DANS L'EAU

(84) Designated Contracting States:
**AT BE CH CY DE DK ES FI FR GB GR IE IT LI LU MC NL PT SE TR**

(30) Priority: **24.07.2000 US 220376 P**

(43) Date of publication of application:
**23.04.2003 Bulletin 2003/17**

(73) Proprietors:
- **Pharmacia & Upjohn Company**
  **Kalamazoo, MI 49007 (US)**
- **Sugen, Inc.**
  **South San Francisco, CA 94080 (US)**

(72) Inventors:
- **GAO, Ping**
  **Portage, MI 49024 (US)**
- **MOROZOWICH, Walter**
  **Kalamazoo, MI 49002 (US)**
- **SHENOY, Narmada**
  **Sunnyvale, CA 94087 (US)**

(74) Representative: **Perry, Robert Edward**
**GILL JENNINGS & EVERY**
**Broadgate House**
**7 Eldon Street**
**London EC2M 7LH (GB)**

(56) References cited:
**WO-A-01/91727**
**WO-A-98/38984**
**WO-A-99/49848**
**US-A- 4 816 247**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

### Field of the Invention

The invention relates to a formulation for extremely water-insoluble compounds. More particularly, the invention relates to a self-emulsifying drug delivery system for extremely water-insoluble, lipophilic drugs.

### Description of Related Technology

In the pharmaceutical industry, a critical aspect of preparing a desirable product is the ability to properly formulate a poorly water-soluble drug, or active agent. Many drugs, including many indolinone analogs, are extremely insoluble in water and, as a result, the oral bioavailability of these drugs is low due to incomplete absorption. For example, some indolinone compounds have a solubility of only 10 nanograms per milliliter in water. Such solubility is generally believed to be too low for efficient oral absorption.

In addition, some formulations of extremely water-insoluble drugs, for example co-solvent based formulations, result in rapid precipitation of the drug upon aqueous dilution of the formulation under conditions simulating the gastrointestinal tract. Accordingly, scientists actively research and develop formulations for dissolving and solubilizing extremely water-insoluble compounds.

One method for administering extremely insoluble active agents is the self-emulsifying drug delivery system. A self-emulsifying drug delivery system is a uniphase liquid or semi-solid, typically comprising an oil and a surfactant, and having an oily nature, which forms an emulsion when contacted with an aqueous environment. Self-emulsifying drug delivery systems are easily administered and easy to manufacture. The self-emulsifying drug delivery systems offer the potential of improved oral absorption of active agents that are difficult to dissolve in aqueous solution.

Examples of extremely water-insoluble active agents are those compounds having a solubility in water of less than 100 micrograms per milliliter of water at room temperature. These extremely water-insoluble drugs can include various types of steroids, anticancer agents, antifungal agents, and antiinfective agents. It particularly would be beneficial to develop a self-emulsifying drug delivery system having suitable components for solubilizing and administering these extremely water-insoluble active agents in order to take advantage of the therapeutic activities of these compounds. Particularly, it would be beneficial to prepare a formulation for compounds in the indolinone class, which have demonstrated promising anticancer activity.

One possible component for a useful formulation is the hydrophilic, miscible polymer polyvinylpyrrolidone ("PVP"). Generally, polyvinylpyrrolidone is chemically compatible with a large variety of excipients. In the formulation art, however, polyvinylpyrrolidone, typically is used as a binder in tablet or pellet formulations. Primarily, the solid form of polyvinylpyrrolidone is incorporated as a dry powder into a blend of excipients to prepare tablet cores or pellets. For example, the literature reports using polyvinylpyrrolidone polymer dissolved in a solvent to improve the release rate of the active substance. See, for example, U.K. Patent No. 1,425,407, published February 18, 1973. Typically, the solvent is evaporated to obtain a tablet formulation in its dry form. Examples of this use of polyvinylpyrrolidone are described in U.S. Patent No. 5,776,495, issued July 7, 1998; U.S. Patent No. 6,027,747, issued February 22, 2000; and International Publication No. WO 97/0,4749, published February 13, 1997.

A less common use of polyvinylpyrrolidone involves suspending, stabilizing or increasing the viscosity of a topical or orally-administered suspension or solution, including emulsions. examples of such use are described in European Patent Publication No. 214501 A2, published March 18, 1987. When used as a suspending or stabilizing agent, the polyvinylpyrrolidone in the composition is present in small amounts, as determined by weight of the composition. Typically, the amount of polyvinylpyrrolidone in suspensions or emulsions ranges from less than about 1 wt.% to about 5 wt.% of the formulation. See, *Handbook of Pharmaceutical Excipients, 2d edition*, American Pharmaceutical Association, 1994, 392-399.

WO 9838984 discloses a formulation comprising 30 mg/ml indolinone, Gelucire™ 44/14, Miglyol™ 812, Cremophor™ EL and oleic acid, and a formulation comprising 30 mg/ml indolinone, Gelucire™ 44/14, Miglyol™ 812, Cremophor™ EL and polyvinyl pyrrolidone, to solubilize indolinone-based compounds.

Polyvinylpyrrolidone also can be incorporated into a coating composition. Typically, in the context of a coating composition, polyvinylpyrrolidone is employed as a thickener. See, for example, International Publication No. WO 97/47285, published December 18, 1997.

To date, no literature has been reported regarding the use of polyvinylpyrrolidone in an orally-administered self-emulsifying drug delivery system, particularly for aiding dissolution of an extremely water-insoluble drug. Moreover, only a limited body of literature reports using polyvinylpyrrolidone at concentrations beyond 5%, by weight. A beneficial formulation would solubilize a sufficient amount of an extremely water-insoluble active agent for therapeutic administration to an individual and would prevent precipitation of the drug under conditions simulated in the gastrointestinal tract.

## SUMMARY OF THE INVENTION

The invention provides a formulation for an extremely water-insoluble active agent. An extremely water-insoluble active agent typically has a solubility in water of less than about 100 micrograms per milliliter at room temperature. The active agent is incorporated in a suitable pharmaceutical vehicle. The vehicle comprises a polyvinylpyrrolidone polymer, a fatty acid, and a surfactant. When dispersed in an aqueous environment, the formulation spontaneously forms an emulsion wherein the active agent is partitioned and remains solubilized in the emulsified oil phase. The self-emulsifying formulation provides a useful dosage form for administering the active agent to provide enhanced bioavailability over conventional dosage forms.

The self-emulsifying formulation is useful for administering extremely water-insoluble active agents, such as active agents having anticancer activity. The formulation is particularly beneficial for administering lipophilic compounds, for example indolinone derivatives and other compounds which are extremely insoluble in water.

The above and other aspects, advantages, and novel features of the invention will become apparent from the following detailed description of the invention.

## DETAILED DESCRIPTION OF THE INVENTION

Therefore, in one aspect, the invention relates to a formulation for an extremely water-insoluble, lipophilic active agent in a vehicle, wherein the vehicle comprises polyvinylpyrrolidone, a fatty acid, and a surfactant. The formulation spontaneously forms an emulsion when dispersed in an aqueous environment. The extremely water-insoluble active agent typically has a solubility of less than 100 micrograms per milliter of water.

In another aspect, the invention relates to a method of preparing a self-emulsifying system containing an extremely water-insoluble active agent. The method comprises combining, the extremely water-insoluble active agent with polyvinylpyrrolidone, either by solubilizing the active agent in polyvinylpyrrolidone directly, typically by first dissolving the polyvinylpyrrolidone in an organic solvent, or by dissolving the active agent in a solution of fatty acid and surfactant, which then is combined with a solution of polyvinylpyrrolidone dissolved in organic solvent.

In another aspect, the formulation can be used in a method to treat a patient in need of treatment with a steroidal, an antifungal, an antibacterial, or an anticancer medicament, by administering a composition comprising the extremely water-insoluble, lipophilic active agent in a vehicle comprising polyvinylpyrrolidone, a fatty acid, and a surfactant. In particular, the method can be used for cancer treatment, comprising the step of administering an anticancer active agent, such as indolinone compounds, in the formulation, either alone or in combination with additional medicament or formulations.

Use of the formulation comprising the extremely water-insoluble, lipophilic active agent in a vehicle comprising polyvinylpyrrolidone, a fatty acid, and a surfactant, for the manufacture of a medicament for therapeutic treatment, such as steroidal, antifungal, antibacterial, or anticancer treatment, also is contemplated herein.

The invention provides a formulation containing an extremely water-insoluble active agent in a pharmaceutically acceptable vehicle. The vehicle, comprises (a) polyvinylpyrrolidone, (b) a fatty acid, and (c) a surfactant. The vehicle solubilizes the extremely water-insoluble drug in a liquid or semi-solid medium to achieve a high concentration. The improved dissolution and dispersion properties of this formulation affords improved bioavailability of the drug.

A particular advantage of the invention includes that the formulation provides high concentration of an extremely water-insoluble active agent. In addition, a self-emulsifying formulation of the invention reduces or eliminates precipitation of the active agent upon dilution of the formulation in simulated gastric fluid (pH 2, 0.01 N HCl). The self-emulsifying system can be easily encapsulated into gelatin capsules and administered orally into humans or mammals.

The incorporation of polyvinylpyrrolidone in the self-emulsifying hydrophobic formulation achieves a high concentration of an extremely water-insoluble active agent in the formulation. For this reason, the formulation is particularly suitable for the extremely water-insoluble active agents such as indolinone compounds, for example 3-[(2,4-dimethylpyrrol-5-yl)methylene]-2-indolinone and 3-[2,4-dimethyl-5-(2-oxo-1,2-dihydroindol-3-ylidenemethyl)-1*H*-pyrrol-3-yl] propionic acid. More particularly, the formulation is useful for the active agent, 3-[(2,4-dimethylpyrrol-5-yl)methylene]-2-indolinone, achieving a concentration of about 30 mg/g of active agent in the formulation. Upon contact with water, the invention generates a microemulsion that promotes excellent dispersion for rapid drug release at 37°C and provides high oral bioavailability.

Polyvinylpyrrolidone is a synthetic polymer formed from linear 1-vinyl-2-pyrrolidinone groups. The use of polyvinylpyrrolidone has not been described previously as an excipient useful in self-emulsifying hydrophobic formulations. The degree of polymerization of the polymer affords polymers of various weights, by which the polyvinylpyrrolidone can be characterized. A polyvinylpyrrolidone useful in the present invention can have a molecular weight of about 2,500

to about 100,000. An increasing molecular weight of the polyvinylpyrrolidone polymer correlates to increasing viscosity, which is expressed as a K value. Polyvinylpyrrolidone polymers are commercially available from BASF Corporation (Parsippany, New Jersey, U.S.A.) under the trade name KOLLIDON™, and generally can be obtained in K values of 12, 15, 17, 25, 30, 60, and 90. The preferred polymers have a molecular weight of about 2,500 to about 20,000, which correspond to lower K values, such as K12 and K25. A sufficient amount of polyvinylpyrrolidone is used to dissolve the desired amount of the active agent. To achieve the full advantage of the present invention, the active agent is dissolved in the vehicle containing polyvinylpyrrolidone. The invention has a unique advantage in that the polyvinylpyrrolidone, which generally is used for preparing solid formulations, such as tablets or pellets, can dissolve an extremely water-insoluble, lipophilic active agent.

The preferred amount of polyvinylpyrrolidone in the formulation is about 5 wt.% to about 40 wt.% of the total formulation. A more preferred amount of polyvinylpyrrolidone in the formulation is about 10 wt.% to about 30 wt.%, and even more preferably about 10 wt.% to about 25 wt.%.

The polyvinylpyrrolidone can be dissolved in a pharmaceutically acceptable solvent to improve dissolution of the active agent. A suitable solvent typically is a pharmaceutically acceptable solvent, for example alcoholic solvents. Suitable solvents include, but are not limited to, ethanol, polyethylene glycol, propylene glycol, and mixtures thereof. The preferred solvent is ethanol.

The dissolution of the polyvinylpyrrolidone in the solvent generally is homogenous and sufficient to dissolve the desired amount of the drug. The amount of polyvinylpyrrolidone dissolved in the solvent generally is in the range of about 0.5 to about 3 parts of polyvinylpyrrolidone per one part of solvent. The amount of solvent preferably ranges from about 5 wt.% to about 30 wt.% based on the total weight of the formulation.

The fatty acid prevents or eliminates phase separation between the components of the formulation. Phase separation can occur when the water content of the formulation is above about 3%. The fatty acid comprises a linear or branched-chain hydrocarbon substituted with one or more carboxylic acid functional groups, and optionally with one or more hydroxy groups. Saturated and unsaturated fatty acids, preferably containing about 6 to about 22 carbon atoms, are suitable for the invention.

A preferred fatty acid is a linear, substantially unbranched fatty acid containing from about 6 to about 18 carbons. Examples of suitable fatty acids include, but are not limited to, hexanoic acid, octanoic acid, nonanoic acid, decanoic acid, lauric acid, linoleic acid, oleic acid, palmitic acid, and the like, or mixtures thereof.

The addition of a fatty acid improves the solubility and permits successful encapsulation of the formulation, typically into soft gelatin capsules (SGCs), hard gelatin capsules (HGCs), or hydroxypropyl methylcellulose (HPMC) capsules, at concentrations of about 30 mg/g of active agent. For 3-[(2,4-dimethylpyrrol-5-yl)methylene]-2-indolinone, the addition of the fatty acid allows preparation of a stable formulation even in the presence of about 5% to about 7% water, by weight of the total formulation, without phase separation or drug precipitation.

The amount of fatty acid preferably comprises about 5 wt.% to about 35 wt.% of the formulation. The formulation more preferably comprises about 5 wt.% to about 25 wt.% fatty acid. An even more preferable amount of the fatty acid is about 5 wt.% to about 15 wt.%.

The surfactant can be any suitable substance that generates emulsion droplets by dispersing the formulation in an aqueous environment. As used herein, the term "emulsion droplets" refers to microscopically dispersed droplets in an aqueous environment, generally having a droplet size of less than or equal to 50 µm, wherein each droplet comprises a surfactant layer surrounding an oil core.

A variety of pharmaceutically acceptable surfactants are suitable for use in the invention. Generally, surfactants suitable for the invention are nonionic surfactants, for example, polyoxylated castor oil, polyoxylated glycerides of fatty acid, polyethylene sorbitan fatty acid esters, polyglycolyzed glycerides, and the like, or mixtures thereof. Examples of surfactants useful for the invention include, polyoxyl 40 hydrogenated castor oil sold under the trade name, among the others, CREMOPHOR™ RH40 (BASF Corporation, Parsippany, NJ, U.S.A.); polyoxyl 35 castdr oil sold under the trade name, CREMOPHOR™ EL or CREMOPHOR™ EL-P (both available from BASF Corporation); polyoxylated glycerol fatty acid esters sold under the trade name SOLUTOL™ HS-15, TAGAT™ TO (Goldschmidt Chemical Corp. Hopewell, Virginia, U.S.A.), and PEGLTCOL™ 6-oleate; polyoxyethylene sorbitan fatty acid esters; polyoxytheylene stearates; saturated polyglycolyzed glyerides; or poloxamers; all of which are commercially available. Polyoxyethylene sorbitan fatty acid esters can include polysorbates, for example, polysorbate 20, polysorbate 40, polysorbate 60, and polysorbate 80. Polyoxyethylene stearates can include polyoxyl 6 stearate, polyoxyl 8 stearate, polyoxyl 12 stearate and polyoxyl 20 stearate. Saturated polyglycolyzed glycerides are, for example, GELUCIRE™ 44/14 or GELUCIRE™ 50/13 (Gattefosse, Westwood, New Jersey, U.S.A.). Poloxamers used herein include poloxamer 124 and poloxamer 188. Each surfactant can be used individually or in combination with other suitable surfactants.

The surfactant generally comprises about 20 wt.% to about 70 wt.% of the total composition. More preferably, the formulation comprises about 30 wt.% to about 50 wt.% surfactant.

The addition of an antioxidant to the composition provides the beneficial advantage of increased shelf life to the product. Any antioxidant compatible with the formulation can be used. The preferred antioxidants retard oxidation

of the active agent in the formulation to provide a stable, effective composition. Preferred antioxidants include, for example, ascorbic acid, ascorbyl palmitate, butylhydroxyanisole, butylhydroxytoluene, propyl gallate, sodium ascorbate, tocopherol, and the like, or mixtures thereof. An antioxidant is incorporated in a suitable amount to oxidize excess ions in the formulation. In a preferred formulation, the antioxidant comprises less than about 1 wt.% of the total formulation and, more preferably, from about 0.05 wt.% to about 0.5 wt.% of the total formulation.

If desired, the formulation may further include conventional pharmaceutical additives. Examples of pharmaceutical additives include, but are not limited to, co-surfactants (for examples, sodium lauryl sulfate), coloring agents, flavoring agents, preserving agents, stabilizers, and/or thickening agents.

The formulation can have a liquid or semi-solid form and, if desired, can be filled into a gelatin capsule. After administration, the capsule ruptures and releases the formulation. When the formulation contacts an aqueous environment, for example in the gastrointestinal tract, the formulation spontaneously forms an emulsion. One advantage of the invention is that active agents having poor water-solubility can be solubilized and formulated into a beneficial therapeutic formulation.

This benefit of the invention is best achieved with extremely water-insoluble active agents having a low solubility of less than 100 micrograms per milliliter of water. The extremely water-insoluble active agents having a log P equal or larger than 2 are considered lipophilic compounds, which are particularly suitable for the invention. The term "log P" refers to the logarithms of the partition coefficient of the drug between two immiscible phases, in this case, n-octanol and water. Examples of active agents suitable for the invention include, but are not limited to, active agents having steroidal, anticancer, antifungal, and antiinfective activity. Nonlimiting examples of compounds suitable for the invention are the extremely water-insoluble active agents, for example, progesterone, ketoconazole, itraconazole, metroxyprogesterone, and paclitaxel. Other compounds suitable for the invention are extremely water-insoluble indolinones. Preferred compounds for the formulation of the invention are disclosed in U.S. Patent No. 5,792,783, issued August 11, 1998 describing 3-heteroaryl-2-indolinone compounds of the formula:

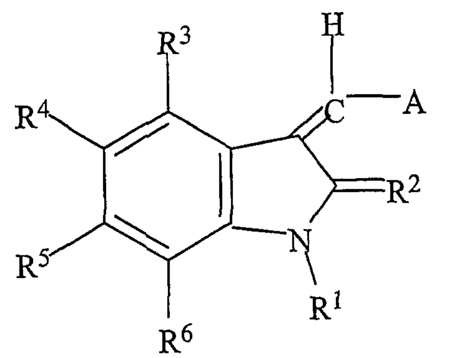

or a pharmaceutically acceptable salt thereof, wherein:

- $R^1$ is H or alkyl;
- $R^2$ is O or S;
- $R^3$, $R^4$, $R^5$, and $R^6$ are each independently selected from the group consisting of hydrogen, alkyl, alkoxy, aryl, aryloxy, alkaryl, alkaryloxy, halogen, trihalomethyl, S(O)R, $SO_2NRR'$, $SO_3R$, SR, $NO_2$, NRR', OH, CN, C(O)R, OC(O)R, NHC(O)R, $(CH_2)_nCO_2R$, and CONRR' ;
- A is a five membered heteroaryl ring selected from the group consisting of thiophene, pyrrole, pyrazole; imidazole, 1,2,3-triazole, 1,2,4-triazole, oxazole, isoxazole, thiazole, isothiazole, 2-sulfonylfuran, 4-alkylfuran, 1,2,3-oxadiazole, 1,2,4-oxadiazole, 1,2,5-oxadiazole, 1,3,4-oxadiazole, 1,2,3,4-oxatriazole, 1,2,3,5-oxatriazole, 1,2,3-thiadiazole, 1,2,4-thiadiazole, 1,2,5-thiadiazole, 1,3,4-thiadiazole, 1,2,3,4-thiatriazole, 1,2,3,5-thiatriazole, and tetrazole, wherein said ring is optionally substituted at one or more positions with alkyl, alkoxy, aryl, aryloxy, alkaryl, alkaryloxy, halogen, trihalomethyl, S(O)R, $SO_2NRR'$, $SO_3R$, SR, $NO_2$, NRR', OH, CN, C(O)R, OC(O)R, NHC(O)R, $(CH_2)_nCO_2R$ or CONRR';
- n is 0-3; and
- R and R' are, independently, H, alkyl or aryl.

As used herein, the term "pharmaceutically acceptable salt" refers to those salts which retain the biological effectiveness and properties of the free bases and which are obtained by reaction with inorganic acids, such as hydrochloric acid, hydrobromic acid, sulfuric acid, nitric acid, phosphoric acid, methanesulfonic acid, ethanesulfonic acid,

p-toluenesulfonic acid, salicylic acid and the like, for example.

The term "alkyl" refers to a straight-chain, branched, or cyclic saturated aliphatic hydrocarbon. Preferably, the alkyl group has 1 to 12 carbons. More preferably, the alkyl group is a lower alkyl having 1 to 7 carbons, more preferably 1 to 4 carbons. Typical alkyl groups include methyl, ethyl, propyl, isopropyl, butyl, isobutyl, tertiary butyl, pentyl, hexyl and the like. The alkyl group can be optionally substituted with one or more substituents selected from the group consisting of hydroxyl, cyano, alkoxy, O, S, $NO_2$, halogen, amino, and SH.

As used herein, the term "alkoxy" refers to an "-O-alkyl" group.

As used herein, the term "aryl" refers to an aromatic group which has at least one ring having a conjugated pi electron system and includes carbocyclic aryl, heterocyclic aryl and biaryl groups. The aryl group can be optionally substituted with one or more substituents selected from the group consisting of halogen, trihalomethyl, hydroxyl, SH, OH, $NO_2$, thioether, cyano (CN), alkoxy, alkyl, and amino.

As used herein, the term "aryloxy" refers to an "-O-aryl" group.

The term "alkaryl" as used herein refers to an alkyl that is covalently joined to an aryl group. Preferably, the alkyl is a lower alkyl.

As used herein, the term "alkylaryloxy" refers to an "-O-alkylaryl" group.

The term "carbocyclic aryl" as used herein refers to an aryl group wherein the ring atoms are carbon.

As used herein, the term "halogen" refers to a bromine, chlorine, fluorine, or iodine atom.

As used herein, the term "heterocyclic aryl" refers to an aryl group having from 1 to 3 heteroatoms as ring atoms, the remainder of the ring atoms being carbon. Heteroatoms include oxygen, sulfur, and nitrogen. Thus, heterocyclic aryl groups include furanyl, thienyl, pyridyl, pyrrolyl, N-lower alkylpyrrolo, pyrimidyl, pyrazinyl, imidazolyl, and the like.

As used herein, the term "amino" refers to a -N($R^a$)$R^b$ group, wherein $R^a$ and $R^b$ are, independently, selected from the group consisting of hydrogen, alkyl, aryl, and alkylaryl.

The more preferred compounds for the formulation are those of formula (I) wherein the substituent A is a pyrrole group optionally substituted with a substituent selected from the group consisting of alkyl, alkoxy, halogen, and -COR, wherein R is as previously defined. Yet more preferred compounds for the formulation are 3-[(2,4-dimethylpyrrol-5-yl)methylene]-2-indolinone, 3-[2,4-dimethyl-5-(2-oxo-1,2-dihydroindol-3-ylidenemethyl)-1*H*-pyrrol-3-yl]propionic acid, and salts thereof. The preferred amount of the active agent is from about 1 wt.% to about 4 wt.% of the formulation.

The formulation allows for the oral administration of extremely water-insoluble active agents to achieve sufficiently high oral bioavailability to treat a disease, condition, or symptom of a disease. The improved formulation is achieved by solubilizing the extremely water-insoluble compound in a solution of polyvinylpyrrolidone dissolved in pharmaceutically acceptable organic solvent, preferably ethanol. The resultihg polyvinylpyrrolidone solution is incorporated into a mixture comprising the fatty acid and the surfactant.

The fatty acid and surfactant are useful excipients for providing a self-emulsifying formulation, which spontaneously forms an emulsion upon conta'ct with an aqueous environment. Generally, conventional usage dictates that polyvinylpyrrolidone is a component of solid, tablet or pellet formulations. The invention provides a beneficial formulation by incorporating the advantages of polyvinylpyrrolidone into a self-emulsifying formulation.

In a formulation of the invention, the solubility of extremely water-insoluble, lipophilic active agents, for example 3-[(2,4-dimethylpyrrol-5-yl)methylene]-2-indolinone can be increased from about 15 mg/g of drug dissolved in a vehicle free of polyvinylpyrrolidone to over 30 mg/g in a vehicle of the present invention, to provide a sufficiently high bioavailability for therapeutic treatment. Although it is particularly difficult to solubilize, 3-[(2,4-dimethylpyrrol-5-yl)methylene]-2-indolinone has demonstrated promising anticancer activity. In a typical aqueous formulation, the solubility of the compound is limited to approximately 10 nanograms per milliliter at room temperature. A benefit of the invention is to prepare a formulation of higher drug concentration for extremely water-insoluble compounds, at concentrations of ~30 mg/ml for 3-[(2,4-dimethylpyrrol-5-yl)methylene]-2-indolinone.

The formulation can be achieved by combining the active agent, polyvinylpyrrolidone, fatty acid, surfactant, and alcoholic solvent to obtain a homogenous formulation. For example, the formulation is prepared by dissolving the active agent in a premixed solution of fatty acid and surfactant, then blending the solution obtained therefrom with a premixed solution of polyvinylpyrrolidone dissolved in alcoholic solvent. The formulation then is stirred until homogeneous. In another example, the active agent can be dissolved in the polyvinylpyrrolidone and stirred with a premixed solution of fatty acid and surfactant until homogenous.

The formulation can be filled into an HPMC capsule or a gelatin capsule, including hard- and soft-shell gelatin capsules. Typically, the gelatin capsule comprises gelatin with an optional amount of plasticizer and other optional excipients. Examples of other excipients include, but are not limited to, dyes, colorants, preservatives, and the like.

Preferably, the formulation contains about 1 part to about 2 parts by weight of fatty acid per 1 part to about 3 parts of polyvinylpyrrolidone. The amount of surfactant in the formulation relative to the polyvinylpyrrolidone ranges from about 1 to about 10 parts by weight per part of PVP. Preferably, about 0.5 to about 3 parts by weight of polyvinylpyrrolidone dissolves in one part by weight of ethanol. The amount of polyvinylpyrrolidone in the formulation is

sufficient to dissolve the desired active agent. A preferred formulation wherein the fatty acid and polyvinylpyrrolidone are in a weight ratio of about 2:1 to about 1:3 (fatty acid: polyvinylpyrrolidone) and the surfactant and polyvinylpyrrolidone are in a weight ratio of about 10:1 to about 1:1 (surfactant: polyvinylpyrrolidone) provides an oily liquid that, when mixed with sufficient amount of aqueous medium, forms an emulsion of the active, agent in oily droplets.

The gelatin capsules typically can be administered orally. The formulation also can be in the form of a liquid or semi-solid solution for oral, parenteral, rectal, or topical application. The preferred dosage form is a liquid contained in a soft-shell gelatin capsule or hard gelatin capsule. The daily dosage and therapeutic regimen of administering the formulation can be determined by one with skill in the art of treating and preventing medical conditions. To provide guidance regarding the use of the invention with respect to the treatment of cancer, the formulation can be administered in an amount from about 0.01 to about 200 milligrams of active agent per square meter of surface area to be treated. Any useful amount of the active agent can be incorporated into the formulation.

When the formulation incorporates an anticancer active agent, the formulation can be used in a method of treating and/or preventing cancer in a patient. The preferred anticancer agent for use in the formulation and method of treating and preventing cancer is an indolinone compound, preferably 3-[(2,4-dimethylpyrrol-5-yl)methylene]-2-indolinone or 3-[2,4-dimethyl-5-(2-oxo-1,2-dihydroindol-3-ylidenemethyl)-1*H*-pyrrol-3-yl]propionic acid. The active agent can be used either alone or co-administered with additional active agents. Examples of active agents suitable for co-administration with a formulation of the present invention include, but are not limited to, vascular endothelial growth factor (VEGF), 5-fluorouracil (5-FU), leucovorin, CAMPTOSAR™ (irinotecan HCl), epirubicin, taxotere, taxol, carboplatin, gemcitabine, cisplatin, oxaliplatin, 5-azacitidine, and other signal transduction inhibitors, such as HERCEPTIN™ (trastazumab) and IRESSA™ (inhibitor of epidermal growth factor receptor tyrosine kinase (EGFR-TK)), as well as other cytostatics, for example matrix metalloproteinase inhibitors (MMPIs), avB3 inhibitors, FITs, and the like. Moreover, it is possible that additional active agents, particularly anticancer active agents, having suitable properties, for example having similar solubility, can be incorporated into the vehicle of the invention.

The invention can be better understood in the context of the following examples.

**Example 1**

Determination of Oral Bioavailability

Formulations A-F, shown below, are described in Table 1, which summarizes the composition of each formulation tested.

Table 1:

| Vehicle Composition of Test Formulations | | | | | | |
|---|---|---|---|---|---|---|
| Vehicle Composition[1] | Amount (mg/g) | | | | | |
| | A reference example | B | C | D reference example | E | F reference example |
| 100% ethanol | 66 | 60 | 80 | 80 | 60 | -- |
| PEG-600 | -- | -- | -- | -- | -- | 100 |
| PVP (PK 12) | 13'4 | 120 | 16'0 | 240 | 180 | -- |
| CREMOPHOR™ EL | 300 | 475 | 100 | -- | 160 | 100 |
| CREMOPHOR™ RH40 | 400 | -- | -- | 460 | -- | -- |
| GELUCIRE™ 44/1,4 | -- | -- | 500 | -- | 470 | 700 |
| GDO/GMO (8:2) | 100 | -- | -- | -- | -- | 100 |

[1] The abbreviations and trade names used herein denote the following: PEG-600 refers to polyethylene glycol having an average of 600 moles of ethylene oxide; PVP (PK 12) refers to polyvinylpyrrolidone having a K value of 12; GDO and GMO refer to glycerol dioleate and glycerol monooleate, respectively, CAPMUL™ MCM (Abitech, Columbus, Ohio, U.S.A.) is the trade name for a mixture of monoglycerides of caprylic and capric acids and MIGLYOL™ 812 (Hüls America, Piscataway, New Jersey, U.S.A.) is a mixed triester of glycerin with caprylic, capric, and stearic acids. The weight percentage is based on the total weight of the composition.

Table 1: (continued)

| Vehicle Composition of Test Formulations | | | | | | |
|---|---|---|---|---|---|---|
| Vehicle Composition[1] | Amount (mg/g) | | | | | |
| | A reference example | B | C | D reference example | E | F reference example |
| Oleic Acid | -- | -- | 150 | -- | 120 | -- |
| Octanoic Acid | -- | 200 | -- | -- | -- | -- |
| CAPMUL™ MCM | -- | 145 | -- | 216 | -- | -- |
| MIGLYOL™ 812 | -- | -- | -- | -- | -- | 100 |
| Tocopherol | -- | -- | -- | 22 | 55 | -- |
| Ascorbyl Palmitate | -- | -- | -- | 22 | 55 | -- |

[1] The abbreviations and trade names used herein denote the following: PEG-600 refers to polyethylene glycol having an average of 600 moles of ethylene oxide; PVP (PK 12) refers to polyvinylpyrrolidone having a K value of 12; GDO and GMO refer to glycerol dioleate and glycerol monooleate, respectively, CAPMUL™ MCM (Abitech, Columbus, Ohio, U.S.A.) is the trade name for a mixture of monoglycerides of caprylic and capric acids and MIGLYOL™ 812 (Hüls America, Piscataway, New Jersey, U.S.A.) is a mixed triester of glycerin with caprylic, capric, and stearic acids. The weight percentage is based on the total weight of the composition.

To prepare the formulations above, 30 mg of 3-[(2,4-dimethylpyrrol-5-yl)methylene]-2-indolinone was dissolved in ethanol, polyethylene glycol, or a mixture thereof, in the amounts, by weight, above. The dissolved active agent was combined with the remaining components of the formulation to obtain formulations A-F, respectively. Three additional formulations were prepared for comparison: micronized active agent in the vehicle of formulation E (150 mg/g); micronized active agent suspended in a mixture of GELUCIRE™ 44/14 and lecithin (150 mg/g); and a 10%, by weight, solution of the active agent in lactose.

Drug concentrations in the blood of the test rats were plotted against the time after the drug is administered through an intravenous (i.v.) or oral route. The AUCs (the Area Under the Plasma Concentration-Time Curve) were recorded and integrated using the trapezoidal rule to calculate the absolute bioavailability as shown in Table 2 below.

$$\text{Absolute bioavailability (\%)} = \frac{(AUC)_{oral}/Dose_{oral}}{(AUC)_{iv}/Dose_{iv}}$$

Male beagle dogs were also selected for the in vivo oral bioavailability study. Each dog in the weight range of 11.5 kg - 17.5 kg was fasted overnight prior to dosing. Each formulation was orally administered to a group of dogs (n=4) at a 10 mg/kg dose. The formulation of high concentration of the active agent, 3-[(2,4-dimethylpyrrol-5-yl)methylene]-2-indolinone (~30 mg/g), was encapsulated in gelatin capsules and administered. Serial blood samples of 2 mL were obtained from the jugular vein at 20 and 40 minutes and 1, 2, 4, 6, 8, 12, and 24 hours after dosing. These blood samples were analyzed using a HPLC assay specific for the compound. The blood concentrations of the compound are plotted against the time and the AUCs were obtained to calculate the absolute bioavailability. The results are reported below in Table 2. The designations A-F in Table 2 denote the formulations as described above in Table 1.

Table 2:

| Comparison of Pharmacokinetics for Various Dosage Forms | | | |
|---|---|---|---|
| Formulation | Dose (mg/kg) | AUC (nM.hr) | Absolute Oral Bioavailability (%) |
| A (30 mg/g) reference example | 10 | 1904±2134 | 10±12 |
| B (30 mg/g) | 10 | 2549±2169 | 13±13 |
| C (30 mg/g) | 10 | 2723±1714 | 15±12 |
| D (30 mg/g) reference example | 10 | 2311±2011 | 12±10 |
| F (30 mg/g) reference example | 10 | 1243±1921 | 6±8 |

Table 2: (continued)

| Comparison of Pharmacokinetics for Various Dosage Forms | | | |
|---|---|---|---|
| Formulation | Dose (mg/kg) | AUC (nM.hr) | Absolute Oral Bioavailability (%) |
| Micronized bulk drug in vehicle E | 10 | 228±340 | 1±1 |
| Micronized bulk drug suspension (150 mg/g) in GELUCIRE™ and Lecithin | 10 | 47±64 | 0.2±0.3 |
| 10% bulk drug in lactose | 50 | 0 | 0 |

As shown in Tables 1 and 2, the self-emulsifying drug delivery systems containing polyvinylpyrrolidone achieved 10% to 15% oral bioavailability of 3-[(2,4-dimethylpyrrol-5-yl)methylene]-2-indolinone. In contrast, the tablet and oil suspension formulations show that the conventional formulations only achieve 0% to 1% oral bioavailability of 3-[(2,4-dimethylpyrrol-5-yl)methylene]-2-indolinone.

**Example 2**

General Methods for Preparing a Self-emulsifying Drug Delivery System for an Extremely Water-insoluble Drug

A mixture of polyvinylpyrrolidone and ethanol was prepared for use in the formulation according to the following steps:

1) Weigh the polyvinylpyrrolidone in a glass flask containing a stir bar, then add the required amount of ethanol (EtOH) into the flask with hand mixing.

2) Cap the flask and heat the flask in a 60°C water bath. Stir the PVP/EtOH solution in the flask until the mixture is homogeneous.

3) Cool the flask to room temperature.
The self-emulsifying formulation was prepared according to the following steps below:

4) Weigh the amount of the excipients listed below into a flask containing a stir bar in the following order:

ascorbyl palmitate;
tocopherol;
oleic acid;
CAPMUL™ MCM;
CREMOPHOR™ RH40;

Then cap the flask.

5) Heat the flask in a 65-70°C water bath. Stir the solution in the flask until the mixture is homogeneous.

6) Add the amount of active agent and cap the flask. Repeat step 5, above, until the mixture is homogeneous.

7) Add the PVP/EtOH pre-prepared mixture and cap the flask. Repeat step 5.

The following formulations G-J were prepared with 3-[(2,4-dimethylpyrrol-5-yl)methylene]-2-indolinone active agent according to the methods above.

Table 3:

| Examples of Vehicle Compositions Containing 3-[(2,4-Dimethylpyrrol-5-yl)methylene]-2-indolinone | | | | |
|---|---|---|---|---|
| Vehicle Composition | Amount of Components (mg/g) | | | |
| | G | H | I | J reference example |
| Active agent | 30 mg/g | 30 mg/g | 30 mg/g | 30 mg/g |

Table 3: (continued)

| Examples of Vehicle Compositions Containing 3-[(2,4-Dimethylpyrrol-5-yl)methylene]-2-indolinone | | | | |
|---|---|---|---|---|
| Vehicle Composition | Amount of Components (mg/g) | | | |
| | G | H | I | J reference example |
| PVP (PK 12) | 210 | 150 | 205 | 205 |
| 100% ethanol | 70 | 50 | 65 | 65 |
| CREMOPHOR™ EL | ' -- | -- | 110 | 130 |
| CREMOPHOR™ RH40 | 460 | 460 | -- | -- |
| GELUCIRE™ 44/14 | -- | -- | 480 | 460 |
| CAPMUL™ MCM | -- | 200 | -- | -- |
| GDO/GMO (8:2) | -- | -- | -- | 100 |
| Oleic Acid | 120 | -- | 100 | -- |
| Octanoic Acid | -- | 100 | -- | -- |
| Tocopherol | 5 | 5 | 5 | 5 |
| Ascorbyl Palmitate | 5 | 5 | 5 | 5 |

## Claims

**1.** A self-emulsifying drug delivery system comprising a mixture of

a water-insoluble, lipophilic active agent which has a solubility of less than 100 µg/ml of water, and a log P of at least 2, wherein log P is the logarithm of the partition coefficient of the active agent between n-octanol and water;
polyvinylpyrrolidone;
a fatty acid;
a surfactant; and
an organic solvent in an amount sufficient to dissolve the polyvinylpyrrolidone.

**2.** The system of claim 1, wherein the weight ratio of the fatty acid to the polyvinylpyrrolidone is 2:1 to 1:3.

**3.** The system of claim 2, wherein the weight ratio of the surfactant to the polyvinylpyrrolidone is 10:1 to 1:1.

**4.** The system of any preceding claim, wherein the polyvinylpyrrolidone has a molecular weight of 2,500 to 100,000.

**5.** The system of claim 4, wherein the polyvinylpyrrolidone has a molecular weight of 2,500 to 20,000.

**6.** The system of any preceding claim, which comprises, by weight thereof, 5 to 40% of the polyvinylpyrrolidone.

**7.** The system of any preceding claim, which comprises, by weight thereof, 5 to 35% of the fatty acid.

**8.** The system of claim 7, which comprises, by weight thereof, 5 to 15% of the fatty acid.

**9.** The system of any preceding claim, wherein the fatty acid contains from 6 to 18 carbon atoms.

**10.** The system of claim 9, wherein the fatty acid is selected from hexanoic acid, octanoic acid, nonanoic acid, decanoic acid, lauric acid, linoleic acid, oleic acid, palmitic acid, and mixtures thereof.

**11.** The system of any preceding claim, wherein the surfactant is selected from polyoxylated castor oil, polyoxylated glycerides of fatty acids, polyoxyethylene sorbitan fatty acid esters, polyglycolysed glycerides, and mixtures thereof.

**12.** The system of claim 11, wherein the surfactant is selected from polyoxyl 35 castor oil and polysorbate 80.

**13.** The system of any preceding claim, which comprises, by weight thereof, 20 to 70% of the surfactant.

**14.** The system of claim 13, which comprises, by weight thereof, 30 to 50% of the surfactant.

**15.** The system of any preceding claim, which comprises by weight thereof, 1 to 4% of the active agent; 5 to 40% of the polyvinylpyrrolidone, 5 to 35% of the fatty acid, and 20 to 70% of the surfactant.

**16.** The system of any preceding claim, wherein the organic solvent is selected from ethanol, polyethylene glycols, propylene glycol, and mixtures thereof.

**17.** The system of any preceding claim, further comprising an antioxidant selected from ascorbic acid, ascorbyl palmitate, butylhydroxyanisole, butylhydroxytoluene, propyl gallate, sodium ascorbate, tocopherol, and mixtures thereof.

**18.** The system of any preceding claim, wherein the active agent is a steroid, anticancer agent, antifungal agent or antiinfective agent.

**19.** The system of claim 18, wherein the active agent is selected from progesterone, ketoconazole, itraconazole, medroxyprogesterone and paclitaxel.

**20.** The system of claim 18, wherein the active agent is an anticancer agent selected from paclitaxel and indoline compounds.

**21.** The system of claim 1, wherein the active agent is a compound of the formula:

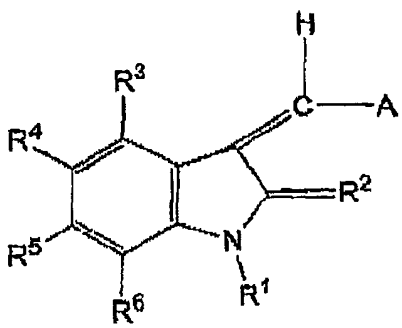

or a pharmaceutically acceptable salt thereof, wherein:

$R^1$ is H or alkyl;
$R^2$ is O or S;
$R^3$, $R^4$, $R^5$, and $R^6$ are each independently selected from the group consisting of hydrogen, alkyl, alkoxy, aryl, aryloxy, alkaryl, alkaryloxy, halogen, trihalomethyl, S(O)R, $SO_2NRR'$, $SO_3R$, SR, $NO_2$, NRR', OH, CN, C(O) R, OC(O)R, NHC (O) R, $(CH_2)_nCO_2R$, and CONRR';
A is a five membered heteroaryl ring selected from the group consisting of thiophene, pyrrole, pyrazole, imidazole, 1,2,3-triazole, 1,2,4-triazole, oxazole, isoxazole, thiazole, isothiazole, 2-sulfonylfuran, 4-alkylfuran, 1,2,3-oxadiazole, 1,2,4-oxadiazole, 1,2,5-oxadiazole, 1,3,4-oxadiazole, 1,2,3,4-oxatriazole, 1,2,3,5-oxatriazole, 1,2,3-thiadiazole, 1,2,4-thiadiazole, 1,2,5-thiadiazole, 1,3,4-thiadiazole, 1,2,3,4-thiatriazole, 1,2,3,5-thiatriazole, and tetrazole, wherein said ring is optionally substituted at one or more positions with alkyl, alkoxy, aryl, aryloxy, alkaryl, alkaryloxy, halogen, trihalomethyl, S(O)R, $SO_2NRR'$, $SO_3R$, SR, $NO_2$, NRR', OH, CN, C (O)R, OC(O)R, NHC(O)R, $(CH_2)_nCO_2R$ or CONRR';
n is 0-3; and
R and R' are each independently H, alkyl or aryl.

**22.** The system of claim 21, wherein the active agent is a compound of formula (I) wherein A is pyrrole optionally substituted with a substituent selected from the group consisting of alkyl, alkoxy, halogen, and -COR.

23. The system of claim 21, wherein the active agent is 3-[(2,4-dimethylpyrrol-5-yl)methylene]-2-indolinone or a salt thereof.

24. The system of claim 21, wherein the active agent is 3- [2,4-dimethyl-5- (2-oxo-1,2-dihydroindol-3-ylidenemethyl)-1H-pyrrol-3-yl] propionic acid or a salt thereof.

25. The system of claim 1, wherein the mixture is filled into a gelatin capsule.

26. The system of claim 25, wherein the gelatin capsule is a hard-shelled gelatin capsule, a soft-shelled gelatin capsule, or a hydroxypropyl methylcellulose capsule.

27. The system of claim 1, wherein the formulation is administered orally, parenterally, rectally, or topically.

28. Use of an active agent as defined in any of claims 18 to 23, for the manufacture of the system of any of claims 18 to 23, for therapy utilising the active agent.

**Patentansprüche**

1. Selbstemulgierendes Arzneimittelabgabesystem, das ein Gemisch von
einem wasserunlöslichen lipophilen Wirkstoff, der eine Löslichkeit von weniger als 100 μg/ml Wasser und einen log P-Wert von mindestens 2 aufweist, wobei log P der Logarithmus des Verteilungskoeffizienten des Wirkstoffs zwischen n-Octanol und Wasser ist;
Polyvinylpyrrolidon;
einer Fettsäure;
einem grenzflächenaktiven Mittel; und
einem organischen Lösemittel in einer zum Lösen des Polyvinylpyrrolidons ausreichenden Menge
umfasst.

2. System nach Anspruch 1, wobei das Gewichtsverhältnis der Fettsäure zu dem Polyvinylpyrrolidon 2:1 bis 1:3 beträgt.

3. System nach Anspruch 2, wobei das Gewichtsverhältnis des grenzflächenaktiven Mittels zu dem Polyvinylpyrrolidon 10:1 bis 1:1 beträgt.

4. System nach einem der vorhergehenden Ansprüche, wobei das Polyvinylpyrrolidon ein Molekulargewicht von 2 500 bis 100 000 aufweist.

5. System nach Anspruch 4, wobei das Polyvinylpyrrolidon ein Molekulargewicht von 2 500 bis 20 000 aufweist.

6. System nach einem der vorhergehenden Ansprüche, das - bezogen auf das Gewicht desselben - 5 bis 40 % an dem Polyvinylpyrrolidon umfasst.

7. System nach einem der vorhergehenden Ansprüche, das - bezogen auf das Gewicht desselben - 5 bis 35 % an der Fettsäure umfasst.

8. System nach Anspruch 7, das - bezogen auf das Gewicht desselben - 5 bis 15 % an der Fettsäure umfasst.

9. System nach einem der vorhergehenden Ansprüche, wobei die Fettsäure 6 bis 18 Kohlenstoffatome enthält.

10. System nach Anspruch 9, wobei die Fettsäure aus Hexansäure, Octansäure, Nonansäure, Decansäure, Laurinsäure, Linolsäure, Ölsäure, Palmitinsäure und Gemischen derselben ausgewählt ist.

11. System nach einem der vorhergehenden Ansprüche, wobei das grenzflächenaktive Mittel aus polyoxyliertem Rizinusöl, polyoxylierten Glyceriden von Fettsäuren, Polyoxyethylensorbitanfettsäureestern, polyglykolysierten Glyceriden und Gemischen derselben ausgewählt ist.

12. System nach Anspruch 11, wobei das grenzflächenaktive Mittel aus Polyoxy-35-Rizinusöl und Polysorbate 80

ausgewählt ist.

**13.** System nach einem der vorhergehenden Ansprüche, das - bezogen auf das Gewicht desselben - 20 bis 70 % an dem grenzflächenaktiven Mittel umfasst.

**14.** System nach Anspruch 13, das - bezogen auf das Gewicht desselben - 30 bis 50 % an dem grenzflächenaktiven Mittel umfasst.

**15.** System nach einem der vorhergehenden Ansprüche, das - bezogen auf das Gewicht desselben - 1 bis 4 % an dem Wirkstoff, 5 bis 40 % an dem Polyvinylpyrrolidon, 5 bis 35 % an der Fettsäure und 20 bis 70 % an dem grenzflächenaktiven Mittel umfasst.

**16.** System nach einem der vorhergehenden Ansprüche, wobei das organische Lösemittel aus Ethanol, Polyethylenglykolen, Propylenglykol und Gemischen derselben ausgewählt ist.

**17.** System nach einem der vorhergehenden Ansprüche, das ferner ein Antioxidationsmittel, das aus Ascorbinsäure, Ascorbylpalmitat, Butylhydroxyanisol, Butylhydroxytoluol, Propylgallat, Natriumascorbat, Tocopherol und Gemischen derselben ausgewählt ist, umfasst.

**18.** System nach einem der vorhergehenden Ansprüche, wobei der Wirkstoff ein Steroid, Antikrebsmittel, Antipilzmittel oder Antiinfektiosum ist.

**19.** System nach Anspruch 18, wobei der Wirkstoff aus Progesteron, Ketoconazol, Itraconazol, Medroxyprogesteron und Paclitaxel ausgewählt ist.

**20.** System nach Anspruch 18, wobei der Wirkstoff ein Antikrebsmittel ist, das aus Paclitaxel und Indolinverbindungen ausgewählt ist.

**21.** System nach Anspruch 1, wobei der Wirkstoff eine Verbindung der Formel:

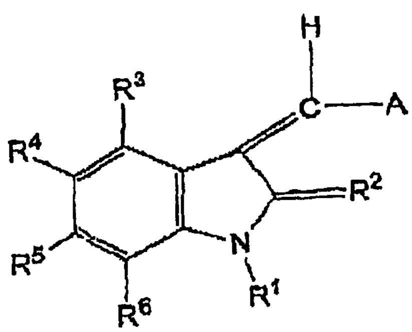

oder ein pharmazeutisch akzeptables Salz derselben ist, worin:

$R^1$ H oder Alkyl bedeutet;
$R^2$ O oder S bedeutet;
$R^3$, $R^4$, $R^5$ und $R^6$ jeweils unabhängig voneinander aus der Gruppe von Wasserstoff, Alkyl, Alkoxy, Aryl, Aryloxy, Alkaryl, Alkaryloxy, Halogen, Trihalogenmethyl, S(O)R, $SO_2NRR'$, $SO_3R$, SR, $NO_2$, NRR', OH, CN, C(O)R, OC(O)R, NHC(O)R, $(CH_2)_nCO_2R$ und CONRR' ausgewählt sind;
A einen fünfgliedrigen Heteroarylring bedeutet, der aus der Gruppe von Thiophen, Pyrrol, Pyrazol, Imidazol, 1,2,3-Triazol, 1,2,4-Triazol, Oxazol, Isoxazol, Thiazol, Isothiazol, 2-Sulfonylfuran, 4-Alkylfuran, 1,2,3-Oxadiazol, 1,2,4-Oxadiazol, 1,2,5-Oxadiazol, 1,3,4-Oxadiazol, 1,2,3,4-Oxatriazol, 1,2,3,5-Oxatriazol, 1,2,3-Thiadiazol, 1,2,4-Thiadiazol, 1,2,5-Thiadiazol, 1,3,4-Thiadiazol, 1,2,3,4-Thiatriazol, 1,2,3,5-Thiatriazol und Tetrazol ausgewählt ist, wobei der Ring optional an einer oder mehreren Positionen mit Alkyl, Alkoxy, Aryl, Aryloxy, Alkaryl, Alkaryloxy, Halogen, Trihalogenmethyl, S(O)R, $SO_2NRR'$, $SO_3R$, SR, $NO_2$, NRR', OH, CN, C(O)R, OC(O)R, NHC(O)R, $(CH_2)_nCO_2R$ oder CONRR' substituiert ist;
n 0 - 3 bedeutet; und

R und R' jeweils unabhängig voneinander H, Alkyl oder Aryl bedeuten.

22. System nach Anspruch 21, wobei der Wirkstoff eine Verbindung der Formel (I), worin A Pyrrol, das optional mit einem Substituenten, der aus der Gruppe von Alkyl, Alkoxy, Halogen und -COR ausgewählt ist, substituiert ist, bedeutet, ist.

23. System nach Anspruch 21, wobei der Wirkstoff 3-[(2,4-Dimethylpyrrol-5-yl)methylen]-2-indolinon oder ein Salz desselben ist.

24. System nach Anspruch 21, wobei der Wirkstoff 3-[(2,4-Dimethyl-5-(2-oxo-1,2-dihydroindol-2-ylidenmethyl)-1Hpyrrol-3-yl]propionsäure oder ein Salz derselben ist.

25. System nach Anspruch 1, wobei das Gemisch in eine Gelatinekapsel gefüllt ist.

26. System nach Anspruch 25, wobei die Gelatinekapsel eine hartschalige Gelatinekapsel, eine weichschalige Gelatinekapsel oder eine Hydroxypropylmethylcellulosekapsel ist.

27. System nach Anspruch 1, wobei die Formulierung oral, parenteral, rektal oder topisch verabreicht wird.

28. Verwendung eines Wirkstoffs gemäß der Definition in einem der Ansprüche 18 bis 23 zur Herstellung des Systems von einem der Ansprüche 18 bis 23 zur Therapie unter Verwendung des Wirkstoffs.

## Revendications

1. Système auto-émulsionnable d'administration de médicament, comprenant un mélange

    d'un agent actif lipophile insoluble dans l'eau, qui a une solubilité inférieure à 100 µg/ml d'eau, et une valeur logP d'au moins 2, logP étant le logarithme du coefficient de partage de l'agent actif entre le n-octanol et l'eau ;
    de polyvinylpyrrolidone ;
    d'un acide gras ;
    d'un agent tensioactif ; et
    d'un solvant organique en une quantité suffisante pour dissoudre la polyvinylpyrrolidone.

2. Système suivant la revendication 1, dans lequel le rapport pondéral de l'acide gras à la polyvinylpyrrolidone est compris dans l'intervalle de 2:1 à 1:3.

3. Système suivant la revendication 2, dans lequel le rapport pondéral de l'agent tensioactif à la polyvinylpyrrolidone est compris dans l'intervalle de 10:1 à 1:1.

4. Système suivant l'une quelconque des revendications précédentes, dans lequel la polyvinylpyrrolidone a un poids moléculaire de 2500 à 100 000.

5. Système suivant la revendication 4, dans lequel la polyvinylpyrrolidone a un poids moléculaire de 2500 à 20 000.

6. Système suivant l'une quelconque des revendications précédentes, qui comprend, en poids de celui-ci, 5 à 40 % de la polyvinylpyrrolidone.

7. Système suivant l'une quelconque des revendications précédentes, qui comprend, en poids de celui-ci, 5 à 35 % de l'acide gras.

8. Système suivant la revendication 7, qui comprend, en poids de celui-ci, 5 à 15 % de l'acide gras.

9. Système suivant l'une quelconque des revendications précédentes, dans lequel l'acide gras contient 6 à 18 atomes de carbone.

10. Système suivant la revendication 9, dans lequel l'acide gras est choisi entre l'acide hexanoïque, l'acide octanoïque, l'acide nonanoïque, l'acide décanoïque, l'acide laurique, l'acide linoléique, l'acide oléique, l'acide palmitique et leurs mélanges.

**11.** Système suivant l'une quelconque des revendications précédentes, dans lequel l'agent tensioactif est choisi entre l'huile de ricin polyoxylatée, des glycérides polyoxylatés d'acides gras, des esters d'acides gras de sorbitanne-polyoxyéthylène, des glycérides polyglycolysés et leurs mélanges.

**12.** Système suivant la revendication 11, dans lequel l'agent tensioactif est choisi entre l'huile de ricin-polyoxyle 35 et le polysorbate 80.

**13.** Système suivant l'une quelconque des revendications précédentes, qui comprend, en poids de celui-ci, 20 à 70 % de l'agent tensioactif.

**14.** Système suivant la revendication 13, qui comprend, en poids de celui-ci, 30 à 50 % de l'agent tensioactif.

**15.** Système suivant l'une quelconque des revendications précédentes, qui comprend, en poids de celui-ci, 1 à 4 % de l'agent actif, 5 à 40 % de la polyvinylpyrrolidone, 5 à 35 % de l'acide gras et 20 à 70 % de l'agent tensioactif.

**16.** Système suivant l'une quelconque des revendications précédentes, dans lequel le solvant organique est choisi entre l'éthanol, des polyéthylèneglycols, le propylèneglycol et leurs mélanges.

**17.** Système suivant l'une quelconque des revendications précédentes, comprenant en outre un antioxydant choisi entre l'acide ascorbique, le palmitate d'ascorbyle, le butylhydroxyanisole, le butylhydroxytoluène, le gallate de propyle, l'ascorbate de sodium, le tocophérol et leurs mélanges.

**18.** Système suivant l'une quelconque des revendications précédentes, dans lequel l'agent actif est un stéroïde, un agent anticancéreux, un agent antifongique ou un agent anti-infectieux.

**19.** Système suivant la revendication 18, dans lequel l'agent actif est choisi entre la progestérone, le kétoconazole, l'itraconazole, la médroxyprogestérone et le paclitaxel.

**20.** Système suivant la revendication 18, dans lequel l'agent actif est un agent anticancéreux choisi entre le paclitaxel et des dérivés d'indoline.

**21.** Système suivant la revendication 1, dans lequel l'agent actif est un composé de formule :

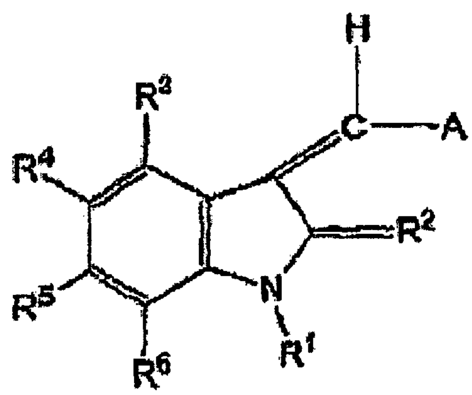

ou un de ses sels pharmaceutiquement acceptables, formule dans laquelle :

$R^1$ représente H ou un groupe alkyle ;
$R^2$ représente un atome de O ou S ;
$R^3$, $R^4$, $R^5$ et $R^6$ sont choisis chacun indépendamment dans le groupe consistant en un atome d'hydrogène, des groupes alkyle, alkoxy, aryle, aryloxy, alkaryle, alkaryloxy, halogéno, trihalogénométhyle, S(O)R, $SO_2NRR'$, $SO_3R$, SR, $NO_2$, NRR', OH, CN, C(O)R, OC(O)R, NHC(O)R, $(CH_2)_nCO_2R$, et CONRR' ;
A représente un noyau hétéroaryle pentagonal choisi dans le groupe consistant en thiophène, pyrrole, pyrazole, imidazole, 1,2,3-triazole, 1,2,4-triazole, oxazole, isoxazole, thiazole, isothiazole, 2-sulfonylfuranne, 4-alkylfuranne, 1,2,3-oxadiazole, 1,2,4-oxadiazole, 1,2,5-oxadiazole, 1,3,4-oxadiazole, 1,2,3,4-oxatriazole, 1,2,3,5-oxatriazole, 1,2,3-thiadiazole, 1,2,4-thiadiazole, 1,2,5-thiadiazole, 1,3,4-thiadiazole, 1,2,3,4-thiatriazole, 1,2,3,5-thiatriazole et tétrazole, ledit noyau étant facultativement substitué à une ou plusieurs positions avec des substituants alkyle, alkoxy, aryle, aryloxy, alkaryle, alkaryloxy, halogéno, trihalogénométhyle, S(O)R, $SO_2NRR'$, $SO_3R$, SR, $NO_2$, NRR', OH, CN, C(O)R, OC(O)R, NHC(O)R, $(CH_2)_nCO_2R$, et CONRR' ;
n a une valeur de 0 à 3 ; et
R et R' représentent chacun indépendamment H, un groupe alkyle ou aryle.

**22.** Système suivant la revendication 21, dans lequel l'agent actif est un composé de formule (I) dans laquelle A représente un noyau pyrrole facultativement substitué avec un substituant choisi dans le groupe consistant en des substituants alkyle, alkoxy, halogéno et -COR.

**23.** Système suivant la revendication 21, dans lequel l'agent actif est la 3-[(2,4-diméthylpyrrol-5-yl)méthylène]-2-indolinone ou un de ses sels.

**24.** Système suivant la revendication 21, dans lequel l'agent actif est l'acide 3-[2,4-diméthyl-5-(2-oxo-1,2-dihydroindol-3-ylidèneméthyl)-1*H*-pyrrol-3-yl]propionique ou un de ses sels.

**25.** Système suivant la revendication 1, dans lequel une capsule de gélatine est remplie avec le mélange.

**26.** Système suivant la revendication 25, dans lequel la capsule de gélatine est une capsule de gélatine à enveloppe dure, une capsule de gélatine à enveloppe molle ou une capsule d'hydroxypropylméthylcellulose.

**27.** Système suivant la revendication 1, dans lequel la formulation est administrée par voie orale, parentérale, rectale ou topique.

**28.** Utilisation d'un agent actif tel que défini dans l'une quelconque des revendications 18 à 23, pour la production du système suivant l'une quelconque des revendications 18 à 23, pour une thérapie au moyen de l'agent actif.